(19) Europäisches Patentamt
European Patent Office
Office européen des brevets

(11) Veröffentlichungsnummer: **0 304 725 B1**

(12) **EUROPÄISCHE PATENTSCHRIFT**

(45) Veröffentlichungstag der Patentschrift: **22.01.92**

(51) Int. Cl.$^5$: **C07C 205/31**, C07C 217/78, C07C 255/54, C07C 323/07, C07C 201/12, C07C 319/14

(21) Anmeldenummer: **88112966.2**

(22) Anmeldetag: **10.08.88**

Die Akte enthält technische Angaben, die nach dem Eingang der Anmeldung eingereicht wurden und die nicht in dieser Patentschrift enthalten sind.

# (54) Verfahren zur Herstellung von Nitro-diphenyl(thio)-ethern.

(30) Priorität: **22.08.87 DE 3728139**

(43) Veröffentlichungstag der Anmeldung:
**01.03.89 Patentblatt 89/09**

(45) Bekanntmachung des Hinweises auf die Patenterteilung:
**22.01.92 Patentblatt 92/04**

(84) Benannte Vertragsstaaten:
**AT BE CH DE FR GB IT LI SE**

(56) Entgegenhaltungen:
**EP-A- 0 022 387**
**US-A- 4 119 629**

**CHEMICAL ABSTRACTS, Band 98, Nr. 15, 11. April 1983, Seite 588, Zusammenfassung Nr. 125524d, Columbus, Ohio, US; A.A. SHTARK et al.: "Interaction of aromatic compounds with nucleophilic reagents in a liquid ammonia medium. II. Nucleophilic substitution in nitrohalo- and polyfluoroaromatic compounds"**

(73) Patentinhaber: **BAYER AG**

**W-5090 Leverkusen 1 Bayerwerk(DE)**

(72) Erfinder: **Hagedorn, Ferdinand, Dr.**
**Domblick 16**
**W-5090 Leverkusen 3(DE)**
Erfinder: **Fiege, Helmut, Dr.**
**Walter-Flex-Strasse 23**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von Nitro-diphenyl(thio)ethern durch Umsetzung von Halogennitrobenzolen und (Thio)Phenolaten in flüssigem Ammoniak, wobei die Reaktion unter Druck und bei einer Temperatur von 0 °C bis 120 °C durchgeführt wird; die Nitrogruppe befindet sich jeweils in ortho- oder para-Stellung zum Ether-Sauerstoff oder Ether-Schwefel bzw. zum Halogen.

Die Herstellung von Nitro-diphenylethern durch Umsetzen von Halogen-nitrobenzolen mit Phenolaten in Reaktionsmedien, wie beispielsweise Chlorbenzol, Phenol, Dimethylsulfoxid, Sulfolan, Dimethylformamid, Dimethylacetamid, N-Methyl-pyrrolidon oder Polyethylenglykol ist bekannt. Bei diesen Umsetzungen wird häufig Kupfer als Katalysator benötigt (Houben-Weyl, Methoden der organischen Chemie, Band VI/3 (Sauerstoffverbindungen I, 1965), S. 86 ff.). Soweit es sich hierbei nicht um besonders leicht reagierende Halogen-nitroverbindungen, wie beispielsweise 2,4-Dinitro-chlorbenzol, handelt, sind allgemein recht hohe Reaktionstemperaturen und lange Reaktionszeiten erforderlich, um überhaupt Umsätze von 60-79 % zu erreichen. Die erwähnten Reaktionsmedien verursachen wegen ihrer aus ökologischer Sicht notwendigen Wiedergewinnung Probleme und finanzielle Aufwendungen.

Die Herstellung von Nitro-diphenylethern ist auch bereits in flüssigem Ammoniak versucht worden. Bei -40 °C wurde in flüssigem Ammoniak selbst bei einer Reaktionsdauer von 10 Stunden noch keine Reaktion von para-Nitrochlorbenzol mit Natriumphenolat beobachtet; selbst das wesentlich stärker aktivierte 2,4-Dinitro-chlorbenzol führt bei der Umsetzung auch mit der doppelten molaren Menge an Natriumphenolat in flüssigem Ammoniak bei -33 °C nur zu einer Ausbeute von 80 % (Zhur Org. Khim. 18 (1982), 2321-2327; englische Übersetzung 1983, 2051-2056).

Es wurde nun überraschend gefunden, daß man Nitro-diphenyl(thio)ether aus Halogen-nitrobenzolen und (Thio)Phenolaten in flüssigem Ammoniak bei höherer Temperatur herstellen kann, als bisher angenommen wurde, obwohl zu befürchten war, daß Halogen-nitrobenzole unter dem Einfluß des in hoher Konzentration vorliegenden Ammoniaks eine Aminolyse zu Nitranilinen eingehen würden. Die Reaktionstemperaturen liegen jedoch beträchtlich unter denen, die in den oben erwähnten Reaktionsmedien bisher für erforderlich gehalten wurden; dies ist vor allem im Hinblick auf die thermische Instabilität mancher Nitroaromaten bei höheren Temperaturen in Gegenwart starker Basen von Bedeutung. Auch der in EP 0 022 387 erwähnte positive Einfluß von basisch beständigen Phasentransferkatalysatoren auf die Reaktionsgeschwindigkeit der Umsetzung von Chlor-nitrobenzolen mit Alkaliphenolaten in den oben erwähnten Reaktionsmedien wird durch die erfindungsgemäße Reaktionsführung noch deutlich übertroffen.

Die Erfindung betrifft demnach ein Verfahren zur Herstellung von Nitro-diphenyl(thio)ethern der Formel

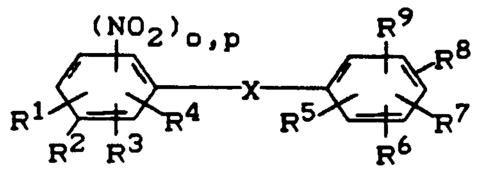 (I),

in der

X für Sauerstoff oder Schwefel steht,

o, p die ortho- oder para-Stellung der Nitrogruppe zum Ether-Sauerstoff oder Ether-Schwefel anzeigt,

$R^1$ und $R^2$ unabhängig voneinander Wasserstoff, Halogen, Nitro, Cyano, Alkyl, Perfluoralkyl oder Alkoxy bedeuten,

$R^3$ und $R^4$ unabhängig voneinander für Wasserstoff, Halogen oder Alkyl stehen,

$R^5$ und $R^6$ unabhängig voneinander Wasserstoff, Halogen, Nitro, Amino, Alkyl, Perfluoralkyl, Alkoxy, Carboxyl, Alkalicarboxlat, Perfluoralkoxy oder Perfluoralkylthio bedeuten,

$R^7$ Wasserstoff, Halogen, X-A, Phenyl-X-A, Alkylphenyl-X-A oder $SO_2$-phenyl-X-A bedeutet, worin

A für ein Äquivalent eines Alkalimetallkations oder für

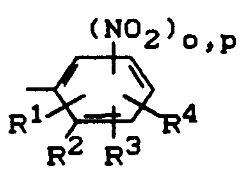

steht, worin o,p und $R^1$ bis $R^4$ die angegebene Bedeutung haben, und

$R^8$ und $R^9$ unabhängig voneinander für Wasserstoff, Halogen oder Alkyl stehen

und worin weiterhin einer der Reste $R^3$ oder $R^4$ auch für

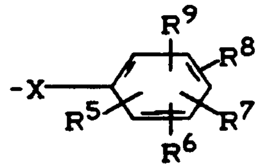

stehen kann, worin X und $R^5$ bis $R^9$ die angegebene Bedeutung haben,
durch Umsetzung von Halogen-nitrobenzolen der Formel

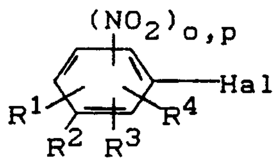

(II),

in der
o, p und $R^1$ bis $R^4$ die angegebene Bedeutung haben,
mit (Thio)Phenolaten der Formel

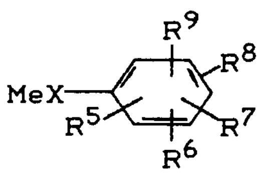

(III),

in der

Me ein Äquivalent eines Alkalimetalls bedeutet und

X und $R^5$ bis $R^9$ die angegebene Bedeutung haben, wobei jedoch Carboxyl als COOMe vorliegt

in flüssigem Ammoniak, das dadurch gekennzeichnet ist, daß man die Reaktion unter Druck und bei einer Temperatur von 0 °C bis +120 °C, bevorzugt von +10 °C bis +110 °C, durchführt und das Ammoniak nach Beendigung der Reaktion durch Destillation abtrennt.

Mindestens eine Nitrogruppe steht in den erfindungsgemäß herstellbaren Nitrodiphenyl(thio)ethern in ortho-oder para-Stellung zum Ether-Sauerstoff oder Ether-Schwefel bzw. in den als Ausgangsstoffen dienenden Halogen-nitrobenzolen in ortho-oder para-Stellung zum Halogensubstituenten, der im Rahmen der erfindungsgemäßen Umsetzung gegen das (Thio)Phenolatanion ausgetauscht wird.

Als Halogen sei beispielsweise Fluor, Chlor, Brom, bevorzugt Chlor oder Brom, besonders bevorzugt Chlor genannt.

Als Alkyl sei der geradkettige oder verzweigte Rest eines aliphatischen Kohlenwasserstoffs verstanden, der in bevorzugter Weise 1-4 C-Atome hat, wie Methyl, Ethyl, Propyl, Isopropyl, Butyl, Isobutyl oder tert.-Butyl; in besonders bevorzugter Weise seien Methyl und Ethyl genannt.

Als Perfluoralkyl sei beispielsweise ein $C_1$-$C_4$-Alkylrest genannt, dessen H-Atome vollständig durch Fluor ersetzt sind, wie Trifluormethyl, Perfluorethyl, Perfluorpropyl oder Perfluorbutyl; in bevorzugter Weise sei Trifluormethyl genannt.

Als Alkoxy sei der Rest eines geradkettigen oder verzweigten aliphatischen Alkohols genannt, der in bevorzugter Weise 1-4 C-Atome hat, wie Methoxy, Ethoxy, Propoxy, Isopropyloxy, Butoxy, Isobutyloxy oder tert.-Butoxy; in bevorzugter Weise seien Methoxy oder Ethoxy genannt. Analoges gilt für Perfluoralkoxy bzw. Perfluoralkylthio.

Der Substituent $R^7$ innerhalb der Formel (I) bzw. (III) kann neben anderen Bedeutungen auch für die über ein Sauerstoff(Schwefel)atom gebundene Gruppe A oder für die über Phenyl-Sauerstoff(Schwefel) gebundene Gruppe A oder für die über Alkyl-phenyl-Sauerstoff(Schwefel) gebundene Gruppe A oder für die

über $SO_2$-Phenyl-Sauerstoff(Schwefel) gebundene Gruppe A stehen, wobei A entweder für ein Äquivalent eines Alkalimetallkations oder für den genannten substituierten Nitrophenylrest steht, der sich durch Entfernung des Hal-Substituierten aus der Formel (II) ergibt.

Für den Fall, daß $R^7$ die genannten Bedeutungen annimmt, ist das (Thio)Phenolat (III) mit einer zweiten Hydroxyl-bzw. Sulfhydrylgruppe ausgestattet (X-A), die entweder ebenso wie die erste (thio)phenolische Gruppe X-Me in der (Thio)Phenolatform oder aber bereits in der veretherten Form vorliegen kann; man gelangt hiermit also zu den Systemen der Dihydroxybenzole, wie gegebenenfalls substituiertes Resorcin, Brenzkatechin oder Hydrochinon, oder zu den entsprechenden Thioverbindungen.

Bei den anderen genannten Bedeutungen für $R^7$ gelangt man in entsprechender Weise zu mehrkernigen Bis(thio)phenolen bzw. ihren Monoethern. Die genannten Bedeutungen leiten sich demnach (in der genannten Reihenfolge) ab von den Systemen Dihydroxydiphenyl, Bis-(hydroxyphenyl)alkyliden-Verbindungen, wie 2,2-Bis-(hydroxyphenyl)-propan (Bisphenol A), Bis-(hydroxyphenyl)-methan, 1,1-Bis-(hydroxyphenyl)-cyclohexan und anderen, sowie dem System des Dihydroxy-diphenyl-sulfons bzw. den entsprechenden Thioverbindungen.

Liegen bei solchen Systemen im Rahmen der Formel (III) beide Hydroxygruppen bzw. Sulfhydrylgruppen in der (Thio)Phenolatform vor, muß die doppelte Menge an Halogen-nitrobenzol der Formel (II) eingesetzt werden, wenn an beiden Hydroxygruppen bzw. Sulfhydrylgruppen eine Etherbildung stattfinden soll.

Selbstverständlich ist es auch möglich, Dihydroxyverbindungen bzw. Disulfhydrylverbindungen der genannten Art einzusetzen, in denen vor der erfindungsgemäßen Umsetzung bereits eine Ethergruppe eingeführt worden war, A also für den genannten Rest steht, der sich durch Entfernung von Hal aus der Formel (II) ergibt.

In entsprechender Weise gilt das vorstehend Gesagte bezüglich der Gruppe A auch für die Formel (I). In bevorzugter Weise hat A im Rahmen der Formel (III) die Bedeutung eines Äquivalents eines Alkalimetalls, während A im Rahmen der Formel (I) bevorzugt für den substituierten Nitrophenylrest steht, der sich durch Entfernung des Hal-Substituenten aus der Formel (II) ergibt, Entsprechendes gilt für $A^1$ bzw. $A^2$ in den weiter unten aufgeführten Formeln (VI) bzw. (VII).

Einer der Reste $R^3$ oder $R^4$ kann neben anderen Bedeutungen auch für die obengenannte Gruppe stehen, die sich durch Entfernung von Me aus der Formel (III) ergibt. Dieser Fall tritt dann ein, wenn mehr als ein Halogen Hal in der Formel (II) durch ortho- oder para-ständiges Nitro aktiviert ist und somit an der Stelle eines solchen weiteren aktivierten Halogens eine weitere Etherbildung stattfindet. In einem solchen Falle muß in der weiter oben geschilderten Form die doppelte Menge an (Thio)Phenolat der Formel (III) zur Reaktion eingesetzt werden, wenn aus einem zweiten aktivierten Halogen eine zweite Etherbindung entstehen soll. In bevorzugter Weise gilt die soeben beschriebene zusätzliche Bedeutung von $R^3$ und $R^4$ im Rahmen der Formel (I), in weniger bevorzugter Weise im Rahmen der Formel (II).

Me bedeutet ein Äquivalent eines Alkalimetalls, beispielsweise $Li^+$, $Na^+$, $K^+$. In bevorzugter Weise seien $Na^+$ und $K^+$ genannt. Es ist erfindungsgemäß zulässig, gemischte (Thio)Phenolate einzusetzen, die verschiedene der genannten Metallkationen enthalten.

In bevorzugter Weise seien Halogen-nitrobenzole der Formel

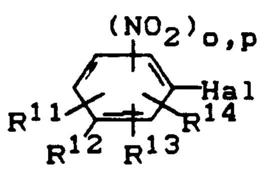

(IV)

genannt, in der

o,p — die ortho- oder para-Stellung der Nitrogruppe zum Halogen anzeigt,

$R^{11}$ — Wasserstoff, Chlor, Nitro, Cyano, Methyl, Ethyl oder Trifluormethyl bedeutet,

$R^{12}$ — Wasserstoff, Chlor, Brom, Methyl, Ethyl, Trifluormethyl, Methoxy oder Ethoxy bedeutet und

$R^{13}$ und $R^{14}$ — unabhängig voneinander für Wasserstoff oder Chlor stehen.

In besonders bevorzugter Weise seien Chlor-nitrobenzole der Formel

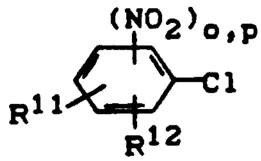

(V)

genannt, in der

- o,p   die ortho- oder para-Stellung der Nitrogruppe zum Chlor anzeigt und
- $R^{11}$ und $R^{12}$   die genannte Bedeutung haben.

In bevorzugter Weise seien (Thio)Phenolate der Formel

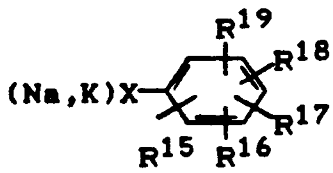

(VI)

genannt, in der

- X   für Sauerstoff oder Schwefel steht,
- $R^{15}$ und $R^{16}$   unabhängig voneinander Wasserstoff, Chlor, Brom, Nitro, Amino, Methyl, Ethyl, Trifluormethyl, Methoxy, Ethoxy, Carboxyl, welches als COONa bzw. COOK vorliegt, Trifluormethoxy oder Trifluormethylthio bedeuten,
- $R^{17}$   Wasserstoff, Chlor, X-$A^1$, Phenyl-X-$A^1$ oder Alkyl-phenyl-X-$A^1$ bedeutet und
- $R^{18}$ und $R^{19}$   unabhängig voneinander für Wasserstoff, Chlor, Methyl oder Ethyl stehen,

wobei $A^1$ für Na, K oder für den Rest

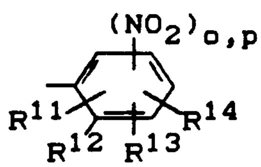

steht, in dem
$R^{11}$ Wasserstoff, Chlor, Nitro, Cyano, Methyl, Ethyl oder Trifluormethyl,
$R^{12}$ Wasserstoff, Chlor, Brom, Methyl, Ethyl, Trifluormethyl, Methoxy oder Ethoxy und
$R^{13}$ und $R^{14}$ unabhängig voneinander Wasserstoff oder Chlor bedeuten.

In besonders bevorzugter Weise seien (Thio)Phenolate der Formel

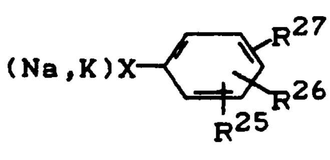

(VII)

genannt, in der

- X   für Sauerstoff oder Schwefel steht,
- $R^{25}$   Wasserstoff, Chlor, Nitro, Amino, Methyl, Ethyl, Trifluormethyl, Methoxy, Ethoxy, Carboxyl, welches als COONa bzw. COOK vorliegt, Trifluormethoxy oder Trifluormethylthio,
- $R^{26}$   Wasserstoff, Chlor, Brom, Methyl, Carboxyl, welches als COONa bzw. COOK vorliegt, oder Trifluormethyl und
- $R^{27}$   Wasserstoff, Chlor oder -X-$A^2$ bedeutet, wobei $A^2$ für Na, K oder für den Rest

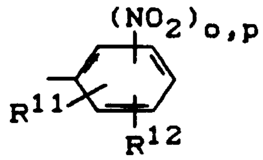

steht, in dem

$R^{11}$ Wasserstoff, Chlor, Nitro, Cyano, Methyl, Ethyl oder Trifluormethyl und

$R^{12}$ Wasserstoff, Chlor, Brom, Methyl, Ethyl, Trifluormethyl, Methoxy oder Ethoxy bedeuten.

In ganz besonders bevorzugter Weise seien (Thio)Phenolate der Formel

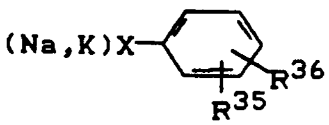

(VIII)

genannt, in der

X für Sauerstoff oder Schwefel steht,

$R^{35}$ Wasserstoff, Chlor, Nitro, Amino, Methyl, Ethyl, Trifluormethyl, Methoxy, Ethoxy, Carboxyl, welches als COONa bzw. COOK vorliegt, Trifluormethoxy oder Trifluormethylthio und

$R^{36}$ Wasserstoff, Chlor, Methyl, Ethyl, Carboxyl, welches als COONa bzw. COOK vorliegt, oder Trifluormethyl bedeuten.

Die erfindungsgemäß möglichen Reaktionen zur (Thio)Etherbildung seien durch folgende Formelgleichungen schematisch dargestellt, wobei das mögliche Substitutionsmuster pauschal durch den nicht bezifferten Buchstaben R dargestellt wird:

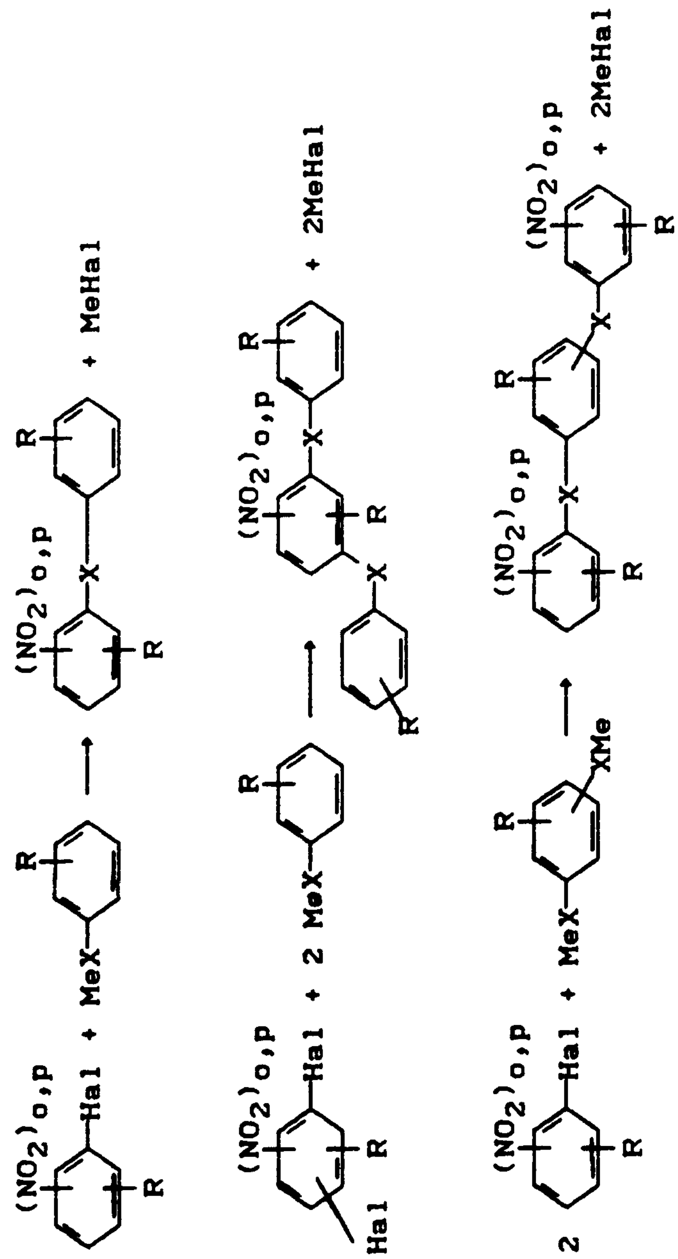

Während in der ersten Formelgleichung die Bildung eines Monoethers angesprochen ist, beziehen sich die zweite und dritte Formelgleichung auf die oben näher beschriebenen Varianten, bei denen mehr als ein aktiviertes Halogen im Halogen-Nitrobenzol vorhanden ist oder mehr als eine Hydroxylgruppe oder Sulfhydrylgruppe in der (Thio)Phenolatform im einzusetzenden (Thio)Phenolat vorhanden ist (in der oben ebenfalls angesprochenen Weise kann bei einer Dihydroxyverbindung (Disulfhydrylverbindung) eine Hydroxygruppe (Sulfhydrylgruppe) vor Eintritt in das erfindungsgemäße Verfahren bereits monoverethert sein bzw. bei mehr als einem aktivierten Halogen eine stufenweise Umsetzung erfolgen).

Geeignete Halogen-Nitrobenzole für das erfindungsgemäße Verfahren sind beispielsweise:

2-Chlor-nitrobenzol, 2-Brom-nitrobenzol, 4-Fluor-nitrobenzol, 4-Chlor-nitrobenzol, 4-Brom-nitrobenzol, 2,3-Dichlor-nitrobenzol, 2,4-Dichlor-nitrobenzol, 2,5-Dichlor-nitrobenzol, 2,6-Dichlor-nitrobenzol, 3,4-Dichlor-nitrobenzol, 2-Chlor-3-nitrotoluol, 6-Chlor-3-nitrotoluol, 1,2,4-Trichlor-5-nitrobenzol, 5-Chlor-2-nitroanilin, 4,6-Dichlor-1,3-dinitrobenzol, 4-Chlor-3-cyano-nitrobenzol, 4-Chlor-2-cyano-nitrobenzol, 5-Chlor-2-nitroanilin, 1,4-Dichlor-2,5-dinitrobenzol.

(Thio)Phenolate für das erfindungsgemäße Verfahren sind beispielsweise die Alkalisalze folgender Hydroxyaromaten:

Phenol, Kresole, Xylenole, Nitrophenole, Chlorphenole, Dichlorphenole, Chlornitrophenole, Aminophenole,

insbesondere meta-Aminophenol, Nitrokresole, Hydrochinon, Resorcin, Brenzkatechin, 4,4′-Dihydroxy-diphenyl, Hexafluor-Bisphenol A, Bisphenol A, Tetramethyl-Bisphenol A, Chlor-trifluormethyl-phenole, insbesondere 2-Chlor-4-(trifluormethyl)-phenol und 2-Chlor-5-(trifluormethyl)phenol, 4-Trifluormethoxy-phenol, 4-Trifluormethylmercapto-phenol, Thiophenole, Mercapto- und Bismercaptoaromaten, Salicylsäure, m- und p-Hydroxybenzoesäure, 3-bzw. 5-Chlor-salicylsäure und Hydroxy-phthalsäuren.

Im erfindungsgemäßen Verfahren wird pro zu bildender Diphenyl(thio)ether-Bindung ein molares Verhältnis von Halogen-nitrobenzol zu (Thio)Phenolatgruppe von 0,7-1,2:1, bevorzugt 0,9-1,05:1, besonders bevorzugt 0,95-1:1 eingestellt.

Das erfindungsgemäße Verfahren wird bei einer Temperatur von -30° C bis +140° C, bevorzugt bei -20° C bis +130° C, besonders bevorzugt bei 0° C bis +120° C, ganz besonders bevorzugt bei +10° C bis +110° C, durchgeführt.

Das eingesetzte Ammoniak wird im Reaktionssystem überwiegend in der flüssigen Phase gehalten. Hierzu wird ein Druck eingestellt, der mindestens dem Dampfdruck aller im Reaktionsgemisch vorhandenen Komponenten entspricht. Darüber hinaus ist es selbstverständlich möglich, durch Aufdrücken von Stickstoff, Edelgasen oder anderen bezüglich des Reaktionssystems inerten Gasen den Druck zu erhöhen. Hierbei werden jedoch keine weiteren Vorteile erzielt. Es ist daher bevorzugt, beim Dampfdruck des gesamten Reaktionssystems zu arbeiten, der sich in Abhängigkeit von der Temperatur einstellt.

Das Ammoniak wird in einer Menge eingesetzt, die 4-12 Mol, bevorzugt 5-10 Mol flüssigen Ammoniaks pro Mol Halogen-nitrobenzol entspricht.

Neben dem als Reaktionsmedium dienenden flüssigen Ammoniak kann ein unter den Verfahrensbedingungen inertes organisches Lösungsmittel mitverwendet werden. Unter den vielen dem Fachmann bekannten inerten Lösungsmitteln sind Benzol, Toluol, Xylol, Chlorbenzol, Dichlorbenzol oder aliphatische oder cycloaliphatische Kohlenwasserstoffe, wie Isododecan, n-Pentan, n-Hexan, Petrolether verschiedener Siedebereiche oder Cyclohexan bevorzugt. Besonders bevorzugt sind Benzol, Toluol, Xylol und aliphatische oder cycloaliphatische Kohlenwasserstoffe. In bevorzugter Form werden solche Lösungsmittel jedoch nur im Rahmen der weiter unten beschriebenen Aufarbeitung eingesetzt.

Das als Reaktionsmedium dienende flüssige Ammoniak hat günstige Lösungsmitteleigenschaften für die im erfindungsgemäßen Verfahren einzusetzenden und entstehenden Stoffe. Dadurch ist es möglich, mit höheren Konzentrationen zu arbeiten, als dies mit den bisher verwendeten Lösungsmitteln möglich war. In Verbindung mit der im allgemeinen kürzeren Reaktionsdauer im Vergleich zu den bisherigen Verfahren erhält man sodann deutlich verbesserte Raum-Zeit-Ausbeuten. Eine zusätzliche Verwendung von Katalysatoren, beispielsweise Kupfer oder Kupferverbindungen, entfällt, so daß keine Schwierigkeiten mit der Entsorgung von Schwermetallabfällen erwachsen. Flüssiges Ammoniak ist ein technisch gut handhabbares, leicht wiedergewinnbares und preiswertes Lösungsmittel, das beispielsweise billiger ist als die weiter oben erwähnten Reaktionsmedien Dimethylsulfoxid, Dimethylformamid, Dimethylacetamid und/oder N-Methylpyrrolidon, wie sie in herkömmlichen Verfahren eingesetzt wurden. Einige von diesen Lösungsmitteln sind zudem bei erhöhter Temperatur nicht vollständig basenstabil und haben einen relativ hohen Siedepunkt, der ihre Wiedergewinnbarkeit erschwert. Zugunsten des erfindungsgemäßen Verfahrens unter Verwendung von flüssigem Ammoniak als Reaktionsmedium ist noch die generell erniedrigte Reaktionstemperatur, verglichen mit den genannten Lösungsmitteln als Reaktionsmedien, anzuführen.

Zur Durchführung des erfindungsgemäßen Verfahrens wird im allgemeinen so verfahren, daß man das (Thio)Phenolat mit verflüssigtem Ammoniak in einem Druckbehälter vorlegt, das Gemisch auf die gewünschte Temperatur erhitzt und bei dieser Temperatur das Halogen-nitrobenzol in dem Maße einbringt, wie die Reaktionswärme abgeführt werden kann.

In einer vorteilhaften und bevorzugten Ausführungsform des Verfahrens wird das (Thio)Phenol mit Alkalihydroxid in flüssigem Ammoniak in das Alkaliphenolat überführt, und in dieses Gemisch wird bei der gewünschten Temperatur das Halogennitrobenzol eingetragen. Hierbei wird im (Thio)Phenol vorhandenes Carboxyl gleichzeitig in Alkalicarboxylat übergeführt. Es muß also eine ausreichende Menge an Alkalihydroxid eingesetzt werden, um sowohl OH (bzw. SH) als auch COOH zu neutralisieren. Gleiches gilt für den Fall, daß eine weitere Hydroxy-bzw. Sulfhydrylgruppe im (Thio)Phenolat (III) vorliegt. Sofern das Halogen-nitrobenzol bei der Reaktionstemperatur flüssig ist, wird es bevorzugt in dieser flüssigen Form in den Druckbehälter eingepumpt. Des weiteren ist auch eine Lösung oder Suspension des Halogen-nitrobenzols in einem unter den Reaktionsbedingungen inerten Lösungsmittel der obengenannten Art für die Zuführung in den Druckbehälter geeignet. Man kan jedoch auch die Ausgangsmaterialien ((Thio)Phenolate und Halogennitrobenzole) als Gemisch vorlegen, sodann flüssiges Ammoniak und gegebenenfalls weiteres inertes Lösungsmittel zusetzen und danach das Reaktionsgemisch auf die gewünschte Reaktionstemperatur bringen. Diese zuletzt genannte Variante empfiehlt sich bei langsam reagierenden Ausgangsmaterialien.

Die Herstellung der (Thio)Phenolate aus der aromatischen Hydroxyverbindung und Alkalihydroxid oder

anderen basischen Alkalimetallverbindungen ist dem Fachmann geläufig. In vielen Fällen ist es vorteilhaft, das (Thio)Phenolat aus diesen Stoffen im Reaktionsgefäß zu bilden.

Die geringe Menge Reaktionswasser stört die (Thio)Etherbildung nicht.

Nach Beendigung der Reaktion wird das Ammoniak abgetrennt, z.B. durch Destillation; es kann ohne weitere Reinigung für einen neuen Ansatz verwendet werden.

Aus dem zurückbleibenden Reaktionsgemisch können die entstehenden anorganischen Salze durch Filtration, Absaugen oder Lösen mit Wasser entfernt werden. Die dabei anfallenden Nitro-diphenyl(thio)ether werden in guter Ausbeute und im allgemeinen in beachtlicher Reinheit erhalten, so daß in manchen Fällen auf eine Nachbehandlung zur Reinigung verzichtet werden kann. Falls noch geringe Verunreinigungen enthalten sind, können diese mit bekannten Methoden, beispielsweise durch Wasserdampfdestillation, Umkristallisieren bzw. Umlösen oder durch Dünnschichtdestillation entfernt werden.

Die beschriebene Aufarbeitung des Reaktionsgemisches kann auch in der Weise variiert werden, daß vor der (völligen) destillativen Rückgewinnung des Ammoniaks ein zusätzliches Lösungsmittel eingesetzt wird. Dieses zusätzliche Lösungsmittel verfolgt den Zweck, eine Gruppe von Stoffen, entweder die anorganischen Salze oder die organischen Anteile des Reaktionsgemisches, in Lösung zu bringen. Demgemäß besteht eine der bevorzugten Aufarbeitungsvarianten darin, ein inertes organisches Lösungsmittel der oben beschriebenen Art zuzusetzen, in welchem das anfallende Alkali- oder Erdalkalihalogenid unlöslich ist. Danach wird das Ammoniak oder das restliche Ammoniak destillativ abgetrennt, das inerte Lösungsmittel mit den gelösten organischen Komponenten von den Salzen befreit, worauf man die Gewinnung der Nitro-diphenyl(thio)ether aus der Lösungsmittelphase nach bekannten Methoden vornimmt. Sofern bereits zur Durchführung der Veretherungsreaktion eine solches inertes organisches Lösungsmittel der genannten Art zugesetzt worden war, kann der weitere Zusatz eines solchen Lösungsmittels zur Aufarbeitung entfallen.

Eine andere bevorzugte Variante der Aufarbeitung nach vorherigem Zusatz eines Lösungsmittels betrifft den Zusatz von Wasser zur Auflösung der anorganischen Salze. Auch hier wird sodann das Ammoniak soweit wie möglich destillativ entfernt, anschließend eine Trennung der wäßrigen und der organischen Phase vorgenommen und die organische Phase nach bekannten Methoden aufgearbeitet.

Erfindungsgemäß herstellbare Nitro-diphenyl(thio)ether sind beispielsweise:
2-Nitro-diphenylether, 4-Nitro-diphenylether, 2-Chlor-4-nitro-diphenylether, 4-Chlor-2-nitro-diphenylether, 4,4′-Dichlor-2-nitro-diphenylether, 2,4′-Dichlor-4-nitro-diphenylether, 4′-Chlor-4-nitro-diphenylether, 4′-Chlor-2-nitro-diphenylether, 2′-Methyl-2-nitro-diphenylether, 3′-Amino-4-nitro-diphenylether, 4,4′-Dinitro-2,2′-dimethyl-diphenylether, 2-Chlor-4-methyl-3′-cyano-4′-nitro-diphenylether, 1,4-Bis-(4′-nitro-phenoxy)-benzol, 5-Chlor-2-nitro-diphenylether, 4-Amino-4′-nitro-diphenylether, 2-Cyano-4-nitro-diphenylether, 4-Methyl-4′-nitro-diphenyl-thioether, 4-Trifluormethoxy-4′-nitro-diphenylether, 4-Trifluormethylthio-4′-nitro-diphenylether, 4-Trifluormethylthio-2′-methyl-4′-nitro-diphenylether, 3-(4′-Nitrophenoxy)-benzoesäure u.a. Es ist dem Fachmann bekannt, in den Nitro-diphenyl(thio)ethern vorhandene Carboxylgruppen mit Hilfe von basischen Alkalimetallverbindungen in die zugehörigen Carboxylatgruppen umzusetzen und umgekehrt aus Carboxylatgruppen durch Behandlung mit Säuren die freien Carboxylgruppen zu bilden.

Die erfindungsgemäß herstellbaren Nitro-diphenyl(thio)ether sind wertvolle Zwischenprodukte zur Herstellung von Farbstoffen, Pflanzenschutzmitteln oder von Polymeren. Die folgenden Beispiele erläutern die Erfindung, ohne sie einzuschränken.

Beispiel 1

157,6 g p-Chlornitrobenzol und 116,1 g Natriumphenolat wurden in einem Stahlautoklaven vorgelegt. Man pumpte bei Raumtemperatur 100 g flüssiges Ammoniak in den Druckbehälter, erhitzte das Gemisch auf 70° und ließ es unter Rühren innerhalb 9 Stunden abreagieren. Der anfangs beobachtete Druck von 28 bar fiel während der Reaktion auf 22 bar. Nach dem Abdestillieren des Ammoniaks und der Aufarbeitung des Destillationsrückstandes erhielt man 4-Nitro-diphenylether mit 213,8 g Ausbeute = 99,4 % der theoretischen Ausbeute. Das Produkt ist 99,2 %ig.

Vergleichsweise benötigte man für diese Reaktion in Chlorbenzol als Reaktionsmedium mit einem basisch beständigen Phasentransferkatalysator gemäß Beispiel 1 der Europ. Patentschrift 22 387 eine Temperatur von 130° bei etwas niedrigerer Ausbeute (95 %). In Abwesenheit des Phasentransferkatalysators betrug die Ausbeute nur 3 %.

Beispiel 2

In einen Stahlautoklaven füllte man 157,6 g o-Chlornitrobenzol und 116,1 g Natriumphenolat. Bei Raumtemperatur pumpte man 150 g flüssiges Ammoniak zu, erhitzte das Gemisch unter Rühren 15

Stunden auf 60° und kühlte es anschließend ab. Nach dem Abdestillieren des Ammoniaks und der Aufarbeitung des Destillationsrückstandes erhielt man 202 g 2-Nitro-diphenylether (94 % der theoretischen Ausbeute), 98,5 %ig.

Vergleichsweise benötigte man für die gleiche Reaktion in Chlorbenzol als Reaktionsmedium anstelle von flüssigem Ammoniak Reaktionstemperaturen bis 150° und eine Gesamtreaktionsdauer von 90 bis 100 Stunden, um ein gleiches Ergebnis zu erzielen.

Beispiel 3

157,6 g p-Chlor-nitrobenzol und 150,5 g Natrium-4-chlorphenolat wurden in einem Stahlautoklaven vorgelegt. Bei Raumtemperatur pumpte man 150 g flüssiges Ammoniak zu, brachte das Gemisch auf 80° C und rührte es bei dieser Temperatur 10 Stunden. Anschließend pumpte man 150 g Wasser zu, kühlte das Gemisch auf Raumtemperatur, entspannte den Druckbehälter unter gleichzeitigem Abdestillieren des Ammoniaks und saugte das Reaktionsgemisch mit einer Nutsche ab. Nach dem Waschen des kristallinen Produkts mit Wasser und nach Trocknen erhielt man 4-Chlor-4′-nitro-diphenylether mit 226,7 g Ausbeute = 92,3 % der theoretischen Ausbeute. Das Produkt ist nahezu 100 %ig.

Beispiel 4

In einen Stahlautoklaven füllte man 1 Mol 3,4-Dichlornitrobenzol und 117 g (1 Mol) 99,1 %iges Natriumphenolat und pumpte bei Raumtemperatur 150 g flüssiges Ammoniak zu. Das Gemisch wurde 2 Stunden bei 50° C gerührt (Druck 21 → 18 bar). Nach dem Abdestillieren des Ammoniaks und dem Aufarbeiten des restlichen Reaktionsgemisches erhielt man 233,9 g 2-Chlor-4-nitro-diphenylether, 99,2 %ig (= 93,7 % der theoretischen Ausbeute).

Beispiel 5

In einen Stahlautoklaven füllte man 128,3 g (0,977 Mol) Natrium-3-amino-phenolat und 153,9 g (0,977 Mol) p-Chlornitrobenzol. Nach Zupumpen von 150 g (8,8 Mol) Ammoniak erhitzte man das Gemisch 12 Stunden auf 70° C unter Rühren. Der Druck betrug ca. 30 bar. Nach Beendigung der Umsetzung pumpte man 200 ml Toluol bei 85° C in das Gemisch, kühlte es ab und entspannte es unter Rühren und gleichzeitigem Abdestillieren des Ammoniaks. Die Toluollösung wurde durch Absaugen vom Salz getrennt. Nach Waschen des Salzes mit Toluol und Entfernen des Lösungsmittels erhielt man 3-Amino-4′-nitro-diphenylether mit einer Ausbeute von 222,7 g = 94,4 % der theoretischen Ausbeute. Der Gehalt ist 98 %.

Beispiele 6 - 16

In gleicher Weise wurden erhalten:
4-Chlor-2-nitro-diphenylether (Fp.: 36-37° C) aus 2,5-Dichlor-nitrobenzol und Na-phenolat;
2-Chlor-4′-nitro-diphenylether aus p-Chlor-nitrobenzol und o-Chlor-Na-phenolat;
4,4′-Dichlor-2-nitro-diphenylether (Fp.: 79° C) aus 2,5-Dichlornitrobenzol und p-Chlor-Na-phenolat;
2,4′-Dichlor-4-nitro-diphenylether (Fp.: 107-108° C) aus 3,4-Dichlornitrobenzol und p-Chlor-Na-phenolat;
4′-Chlor-2-nitro-diphenylether (Fp.: 44-45° C) aus o-Chlor-nitrobenzol und p-Chlor-Na-phenolat;
2′-Methyl-2-nitro-diphenylether (Kp.: 194-196°/19 mbar) aus o-Chlor-nitrobenzol und 2-Methyl-Na-phenolat;
4,4′-Dinitro-2,2′-dimethyl-diphenylether (Fp.: 270° C) aus 3-Methyl-4-chlor-nitrobenzol und 2-Methyl-4-nitro-Na-phenolat;
1,4-Bis-(4′-nitro-phenoxy)-benzol (Fp.: 232-234° C) aus 2 Mol p-Chlornitrobenzol und 1 Mol des Dinatriumsalzes von p-Hydrochinon;
5-Chlor-2-nitrodiphenylether (Fp.: 85° C) aus 2,4-Dichlornitrobenzol und Natriumphenolat;
4-Amino-4′-nitro-diphenylether (Fp.: 130-131° C) aus p-Nitrochlorbenzol und p-Aminophenol-Natrium;
2-Cyano-4-nitro-diphenylether (Fp.: 125-126° C) aus 2-Cyano-4-nitro-chlorbenzol und Natriumphenolat.

Beispiel 17

In einen Stahlautoklaven füllte man 94 g (1 Mol) Phenol und 40 g Natriumhyroxid-Plätzchen (1 Mol), ferner wurden nach Verschließen des Autoklaven 150 g Ammoniak aufgepreßt. Bei 70° C pumpte man 157,5 g (1 Mol) p-Nitrochlorbenzol in flüssiger Form innerhalb einer Stunde in den Autoklaven; das Gemisch wurde weitere 10 Stunden nachgerührt. Nach dem Entspannen sowie dem Waschen des erhaltenen

Produktes mit Wasser wurde der 4-Nitrodiphenylether mit über 99 %iger Reinheit (gaschromatographisch) isoliert.

Beispiel 18

In einen Druckbehälter füllte man 157,5 g (1 Mol) p-Nitrochlorbenzol, 116 g (1 Mol) Natriumphenolat, 50 g Xylol (Isomerengemisch), ferner wurden nach dem Verschließen 100 g Ammoniak aufgedrückt. Das Gemisch wurde 9 Stunden bei 70 °C gerührt (Druck: 22 bar). Nach dem Abkühlen und Entspannen des Druckbehälters wurde das Gemisch mit 250 ml Wasser ausgeschüttelt und die abgetrennte organische Phase analysiert. Sie enthielt 67,8 % 4-Nitrodiphenylether, 30,6 % p-Nitrochlorbenzol und 0,9 % Phenol.

Beispiel 19

In einen Stahlautoklaven füllte man 91,3 g 2-Cyano-4-nitrochlorbenzol (0,5 Mol), 58 g Natriumphenolat (ca. 0,5 Mol) und 100 g fl. $NH_3$. Das Gemisch wurde 10 Stunden bei 16 bis 18 °C gerührt (Druck: 8,8 bar). Nach dem Aufarbeiten des erhaltenen Produkts durch Waschen mit Wasser und Trocknen erhielt man 91,2 %igen 2-Cyano-4-nitro-diphenylether (109,7 g).

Beispiel 20

66 g (0,5 Mol) Hydrochinon-mononatriumsalz, 78,5 g p-Nitrochlorbenzol und 100 g fl. $NH_3$ wurden in einen Stahlautoklaven gefüllt und 6 Stunden bei 80 °C unter Rühren erhitzt. Nach Entspannen und Entfernen des Ammoniaks trug man das erhaltene Produkt in Wasser ein, filtrierte es, wusch es salzfrei und trocknete es: Man erhielt 95 g, Fp. 153 bis 175 °C. Die Substanz enthielt zu 44 % 4-Hydroxy-4′-nitro-diphenylether und zu 55 % 1,4-Bis-(4′-nitro-phenoxy)-benzol.

Beispiel 21

145 g Natrium-4-methyl-thiophenolat und 156,5 g p-Nitrochlorbenzol wurden in einem Stahlautoklaven vorgelegt. Man preßte 150 g Ammoniak auf, erwärmte das Gemisch unter Rühren auf 70 °C und ließ es innerhalb 6 Stunden bei dieser Temperatur abreagieren. Nach dem Entspannen des Druckbehälters, Aufnehmen des Produktes in Wasser, Auswaschen des Salzes und Trocknen erhielt man 241 g 4-Methyl-4′-nitro-diphenyl-thioether, 99,2 %ig (gaschromatographisch), Fp. 80 - 82 °C.

Beispiel 22

89,1 g 4-Trifluormethoxy-phenol (0,5 Mol), 20,0 g Natriumhydroxid und 78,8 g 4-Chlornitrobenzol wurden in einem Stahlautoklaven (0,7 l) vorgelegt und nach Aufpressen von 100 g wasserfreiem Ammoniak 8 Stunden bei 80 °C erhitzt. Nach dem Abkühlen und Entspannen nahm man das Produktgemisch in 400 ml Methylenchlorid auf, trennte das Ungelöste ab, wusch die organische Phase mit verdünnter Natronlauge und Wasser, trocknete sie und destillierte das Lösungsmittel ab. Der zunächst flüssige Rückstand kristallisierte beim Erkalten und konnte aus Alkohol umgelöst werden. Fp. 41,5 °C. Ausbeute: 98 % d.Th.

Beispiele 23 u. 24

In gleicher Weise wurden erhalten:
4-Trifluormethylmercapto-4′-nitro-diphenylether, (Fp. 41-42 °C), aus 4-Trifluormethylmercapto-Na-phenolat und 4-Chlor-nitrobenzol;
4-Trifluormethylmercapto-2′-methyl-4′-nitrodiphenylether, (Fp. 63-64 °C), aus 4-Trifluormethylmercapto-Na-phenolat und 6-Chlor-2-nitro-toluol.

Beispiel 25

88,5 g (0,635 Mol) 3-Hydroxy-benzoesäure, 99 %ig, 100 g p-Nitrochlorbenzol und 51,4 g Natriumhydroxid (98,8 %ig) wurden in einen Autoklav gefüllt. Nach Entfernen der Luft durch Evakuieren wurden 150 g Ammoniak aufgedrückt. Das Gemisch wurde 10 Stunden bei 80 °C gerührt. Der Druck fiel dabei von 37 auf 34 bar. Nach dem Abkühlen und Entspannen des Ammoniaks löste sich das erhaltene Produkt in 800 ml Wasser. Durch Ansäuren mit Salzsäure fällte man die rohe 3-(4′-Nitrophenoxy)-benzoesäure aus.

Roh-Gehalt: 97,3 %. Durch Umlösen gelang die Reinigung. Ausbeute: 94 %, Fp. 188 °C.

## Patentansprüche

**1.** Verfahren zur Herstellung von Nitro-diphenyl(thio)-ethern der Formel

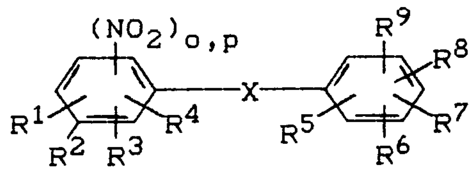

,

in der

| | |
|---|---|
| X | für Sauerstoff oder Schwefel steht, |
| o, p | die ortho- oder para-Stellung der Nitrogruppe zum Ether-Sauerstoff oder Ether-Schwefel anzeigt, |
| $R^1$ und $R^2$ | unabhängig voneinander Wasserstoff, Halogen, Nitro, Cyano, Alkyl, Perfluoralkyl oder Alkoxy bedeuten, |
| $R^3$ und $R^4$ | unabhängig voneinander für Wasserstoff, Halogen oder Alkyl stehen, |
| $R^5$ und $R^6$ | unabhängig voneinander Wasserstoff, Halogen, Nitro, Amino, Alkyl, Perfluoralkyl, Alkoxy, Carboxyl, Alkalicarboxylat, Perfluoralkoxy oder Perfluoralkylthio bedeuten, |
| $R^7$ | Wasserstoff, Halogen, X-A, Phenyl-X-A, Alkyl-phenyl-X-A oder $SO_2$-phenyl-X-A bedeutet, worin |
| A | für ein Äquivalent eines Alkalimetallkations oder für 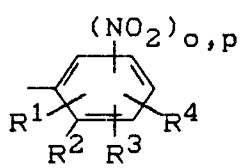 steht, worin o, p und $R^1$ bis $R^4$ die angegebene Bedeutung haben, und |
| $R^8$ und $R^9$ | unabhängig voneinander für Wasserstoff, Halogen oder Alkyl stehen |

und worin weiterhin einer der Reste $R^3$ oder $R^4$ auch für

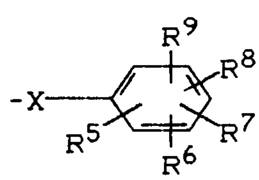

stehen kann, worin X und $R^5$ bis $R^9$ die angegebene Bedeutung haben,,

durch Umsetzung von Halogen-nitrobenzolen der Formel

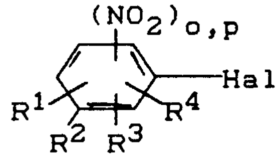

,

in der

o, p und $R^1$ bis $R^4$ die angegebene Bedeutung haben,

mit (Thio)Phenolaten der Formel

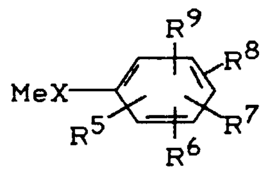

,

in der

| | |
|---|---|
| Me | ein Äquivalent eines Alkalimetalls bedeutet und |
| X und $R^5$ bis $R^9$ | die angegebene Bedeutung haben, wobei jedoch Carboxyl als COOMe vorliegt, |

in flüssigem Ammoniak, dadurch gekennzeichnet, daß man die Reaktion unter Druck und bei einer Temperatur von 0 °C bis +120 °C, bevorzugt von +10 °C bis +110 °C, durchführt und das Ammoniak nach Beendigung der Reaktion durch Destillation abtrennt.

**2.** Verfahren nach Anspruch 1, dadurch gekennzeichnet, daß man Halogen-nitrobenzole der Formel

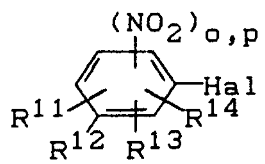

umsetzt, in der

| | |
|---|---|
| o,p | die ortho- oder para-Stellung der Nitrogruppe zum Halogen anzeigt, |
| $R^{11}$ | Wasserstoff, Chlor, Nitro, Cyano, Methyl, Ethyl oder Trifluormethyl bedeutet, |
| $R^{12}$ | Wasserstoff, Chlor, Brom, Methyl, Ethyl, Trifluormethyl, Methoxy oder Ethoxy bedeutet und |
| $R^{13}$ und $R^{14}$ | unabhängig voneinander für Wasserstoff oder Chlor stehen. |

**3.** Verfahren nach Anspruch 1, dadurch gekennzeichnet, daß man Chlor-nitrobenzole der Formel

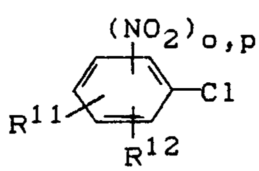

umsetzt, in der

| | |
|---|---|
| o,p | die ortho- oder para-Stellung der Nitrogruppe zum Chlor anzeigt, |
| $R^{11}$ | Wasserstoff, Chlor, Nitro, Cyano, Methyl, Ethyl oder Trifluormethyl und |
| $R^{12}$ | Wasserstoff, Chlor, Brom, Methyl, Ethyl, Trifluormethyl, Methoxy oder Ethoxy bedeuten. |

**4.** Verfahren nach Anspruch 1, dadurch gekennzeichnet, daß man (Thio)Phenolate der Formel

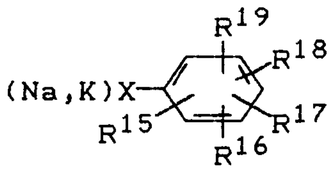

umsetzt, in der

X für Sauerstoff oder Schwefel steht,
$R^{15}$ und $R^{16}$ unabhängig voneinander Wasserstoff, Chlor, Brom, Nitro, Amino, Methyl, Ethyl, Trifluormethyl, Methoxy oder Ethoxy, Carboxyl, welches als COONa bzw. COOK vorliegt, Trifluormethoxy oder Trifluormethylthio bedeuten,
$R^{17}$ Wasserstoff, Chlor, X-$A^1$, Phenyl-X-$A^1$ oder Alkyl-phenyl-X-$A^1$ bedeutet und
$R^{18}$ und $R^{19}$ unabhängig voneinander für Wasserstoff, Chlor, Methyl oder Ethyl stehen,

wobei $A^1$ für Na, K oder für den Rest

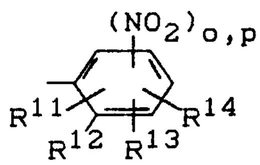

steht, in dem

$R^{11}$ Wasserstoff, Chlor, Nitro, Cyano, Methyl, Ethyl oder Trifluormethyl,

$R^{12}$ Wasserstoff, Chlor, Brom, Methyl, Ethyl, Trifluormethyl, Methoxy oder Ethoxy und

$R^{13}$ und $R^{14}$ unabhängig voneinander Wasserstoff oder Chlor bedeuten.

**5.** Verfahren nach Anspruch 1, dadurch gekennzeichnet, daß man (Thio)Phenolate der Formel

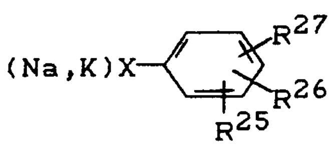

umsetzt, in der

X für Sauerstoff oder Schwefel steht,
$R^{25}$ Wasserstoff, Chlor, Nitro, Amino, Methyl, Ethyl, Trifluormethyl, Methoxy, Ethoxy, Carboxyl, welches als COONa bzw. COOK vorliegt, Trifluormethoxy oder Trifluormethylthio
$R^{26}$ Wasserstoff, Chlor, Brom, Methyl, Ethyl, Carboxyl, welches als COONa bzw. COOK vorliegt, oder Trifluormethyl und
$R^{27}$ Wasserstoff, Chlor oder -X-$A^2$ bedeutet,

wobei $A^2$ für Na, K oder für den Rest

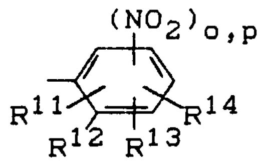

steht, in dem

$R^{11}$ Wasserstoff, Chlor, Nitro, Cyano, Methyl, Ethyl oder Trifluormethyl und

$R^{12}$ Wasserstoff, Chlor, Brom, Methyl, Ethyl, Trifluormethyl, Methoxy oder Ethoxy bedeuten,

6. Verfahren nach Anspruch 1, dadurch gekennzeichnet, daß man pro zu bildender Diphenyl(thio)ether-Bindung ein molares Verhältnis von Halogen-nitrobenzol zu (Thio)Phenolatgruppe von 0,7-1,2:1, bevorzugt 0,9-1,05:1, besonders bevorzugt 0,95-1:1 einstellt.

7. Verfahren nach Anspruch 1, dadurch gekennzeichnet, daß man pro Mol Halogen-nitrobenzol 4-12 Mol flüssiges Ammoniak, bevorzugt 5-10 Mol flüssiges Ammoniak, einsetzt.

8. Verfahren nach Anspruch 1, dadurch gekennzeichnet, daß man vor der (völligen) destillativen Abtrennung des Ammoniaks dem Reaktionsgemisch ein inertes Lösungsmittel, bevorzugt Benzol, Toluol, Xylol, Chlorbenzol, Dichlorbenzol oder einen aliphatischen Kohlenwasserstoff, wie Cyclohexan oder Isododecan, besonders bevorzugt Benzol, Toluol, Xylol oder einen aliphatischen oder cycloaliphatischen Kohlenwasserstoff, zufügt, nach dem Entfernen des Ammoniaks das Alkalihalogenid abtrennt und das Reaktionsprodukt in bekannter Weise aus dem inerten Lösungsmittel gewinnt.

9. Verfahren nach Anspruch 1, dadurch gekennzeichnet, daß man nach der destillativen Abtrennung eines Teils des Ammoniaks dem Reaktionsgemisch Wasser zusetzt, nach dem Entfernen des restlichen Ammoniaks die organische Phase von der wäßrigen abtrennt und die organische Phase zur Gewinnung des Reaktionsproduktes in bekannter Weise aufarbeitet.

10. Verfahren nach Anspruch 1, dadurch gekennzeichnet, daß das (Thio)Phenolat im Reaktionsgemisch aus (Thio)Phenol und Alkalimetallhydroxid gebildet wird, wobei vorhandenes Carboxyl gleichzeitig in Alkalicarboxylat übergeführt wird.

## Claims

1. Process for preparing nitrodiphenyl (thio)ethers of the formula

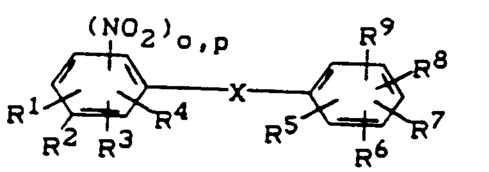

,

in which

X represents oxygen or sulphur,

o, p indicates the ortho- or para-position of the nitro group with respect to the ether oxygen or ether sulphur,

$R^1$ and $R^2$ independently of one another denote hydrogen, halogen, nitro, cyano, alkyl, perfluoroalkyl or alkoxy,

$R^3$ and $R^4$ independently of one another represent hydrogen, halogen or alkyl,

$R^5$ and $R^6$ independently of one another denote hydrogen, halogen, nitro, amino, alkyl, perfluoroalkyl, alkoxy, carboxyl, alkali metal carboxylate, perfluoroalkoxy or perfluoroalkylthio,

$R^7$ denotes hydrogen, halogen, X-A, phenyl-X-A, alkylphenyl-X-A or $SO_2$-phenyl-X-A, in

which A represents an equivalent of an alkali metal cation or represents

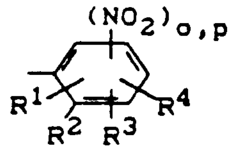

where o, p and $R^1$ to $R^4$ have the meaning mentioned and

$R^8$ and $R^9$ independently of one another represent hydrogen, halogen or alkyl

and in which furthermore one of the radicals $R^3$ or $R^4$ can also represent

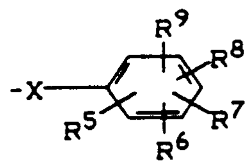

where X and $R^5$ to $R^9$ have the meaning mentioned, by reacting halogenonitrobenzenes of the formula

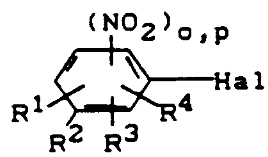

in which

o, p and $R^1$ to $R^4$ have the meaning mentioned with (thio)phenolates of the formula

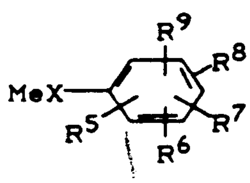

in which

Me denotes an equivalent of an alkali metal and

X and $R^5$ to $R^9$ have the meaning mentioned, carboxyl being present, however, as COOMe,

in liquid ammonia, characterized in that the reaction is carried out under pressure and at a temperature from 0°C to +120°C, preferably from +10°C to +110°C, and the ammonia is separated off by distillation after the reaction is complete.

**2.** Process according to Claim 1, characterized in that halogenonitrobenzenes of the formula

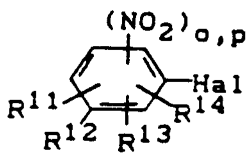

in which

| | |
|---|---|
| o, p | indicates the ortho- or para-position of the nitro group with respect to halogen, |
| $R^{11}$ | denotes hydrogen, chlorine, nitro, cyano, methyl, ethyl or trifluoromethyl, |
| $R^{12}$ | denotes hydrogen, chlorine, bromine, methyl, ethyl, trifluoromethyl, methoxy or |

ethoxy and
$R^{13}$ and $R^{14}$ independently of one another represent hydrogen or chlorine,
are reacted.

**3.** Process according to Claim 1, characterized in that chloronitrobenzenes of the formula

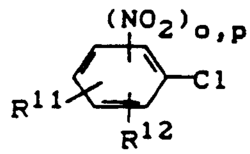

in which
o, p indicates the ortho- or para-position of the nitro group with respect to the chlorine,
$R^{11}$ denotes hydrogen, chlorine, nitro, cyano, methyl, ethyl or trifluoromethyl and
$R^{12}$ denotes hydrogen, chlorine, bromine, methyl, ethyl, trifluoromethyl, methoxy or ethoxy,
are reacted.

**4.** Process according to Claim 1, characterized in that (thio)phenolates of the formula

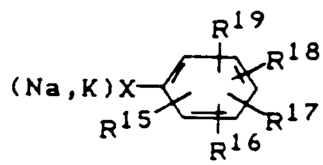

in which
X represents oxygen or sulphur,
$R^{15}$ and $R^{16}$ independently of one another denote hydrogen, chlorine, bromine, nitro, amino, methyl, ethyl, trifluoromethyl, methoxy or ethoxy, carboxyl which is present as COONa or COOK, trifluoromethoxy or trifluoromethylthio,
$R^{17}$ denotes hydrogen, chlorine, X-$A^1$, phenyl-X-$A^1$ or alkylphenyl-X-$A^1$ and
$R^{18}$ and $^{19}$ independently of one another represent hydrogen, chlorine, methyl or ethyl,
where $A^1$ represents Na, K or represents the radical

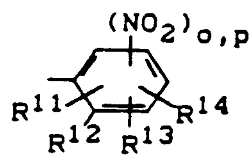

in which $R^{11}$ denotes hydrogen, chlorine, nitro, cyano, methyl, ethyl or trifluoromethyl, $R^{12}$ denotes hydrogen, chlorine, bromine, methyl, ethyl, trifluoromethyl, methoxy or ethoxy and $R^{13}$ and $R^{14}$ independently of one another denote hydrogen or chlorine,
are reacted.

**5.** Process according to Claim 1, characterized in that (thio)phenolates of the formula

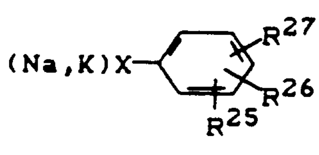

in which
X represents oxygen or sulphur,

$R^{25}$ denotes hydrogen, chlorine, nitro, amino, methyl, ethyl, trifluoromethyl, methoxy, ethoxy, carboxyl which is present as COONa or COOK, trifluoromethoxy or trifluoromethylthio,

$R^{26}$ denotes hydrogen, chlorine, bromine, methyl, ethyl, carboxyl which is present as COONa or COOK, or trifluoromethyl and

$R^{27}$ denotes hydrogen, chlorine or X-$A^2$,
where $A^2$ represents Na, K or represents the radical

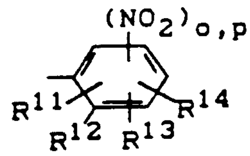

in which
$R^{11}$ denotes hydrogen, chlorine, nitro, cyano, methyl, ethyl or trifluoromethyl and
$R^{12}$ denotes hydrogen, chlorine, bromine, methyl, ethyl, trifluoromethyl, methoxy or ethoxy,
are reacted.

**6.** Process according to Claim 1, characterized in that for each diphenyl (thio)ether bond to be formed a molar halogenonitrobenzene to (thio)phenolate group ratio of 0.7-1.2:1, preferably 0.9-1.05:1, particularly preferably 0.95-1:1 is established.

**7.** Process according to claim 1, characterized in that for each mole of halogenonitrobenzene 4-12 moles of liquid ammonia, preferably 5-10 moles of liquid ammonia, are used.

**8.** Process according to Claim 1, characterized in that before the (complete) removal of ammonia from the reaction mixture by distillation an inert solvent, preferably benzene, toluene, xylene, chlorobenzene, dichlorobenzene or an aliphatic hydrocarbon such as cyclohexane or isododecane, particularly preferably benzene, toluene, xylene or an aliphatic or cycloaliphatic hydrocarbon, is added, after the removal of the ammonia the alkali metal halide is separated off and the reaction product is recovered from the inert solvent in a known manner.

**9.** Process according to Claim 1, characterized in that after the removal of some of the ammonia by distillation water is added to the reaction mixture, after the removal of the remaining ammonia the organic phase is separated from the aqueous phase and the organic phase is worked up in a known manner to recover the reaction product.

**10.** Process according to Claim 1, characterized in that the (thio)phenolate is formed in the reaction mixture from (thio)phenol and an alkali metal hydroxide, any carboxyl present being simultaneously converted into alkali metal carboxylate.

## Revendications

**1.** Procédé de production de (thio)éthers nitro-diphényliques de formule

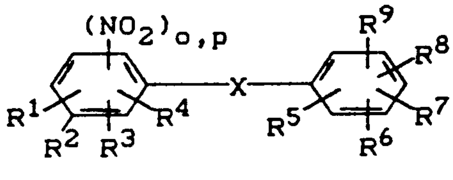

,

dans laquelle
X représente l'oxygène ou le soufre,
o, p représentent la position ortho ou la position para du groupe nitro par rapport à l'oxygène d'éther ou au soufre d'éther,

$R^1$ et $R^2$ représentent, indépendamment l'un de l'autre, l'hydrogène, un halogène, un groupe nitro, cyano, alkyle, perfluoralkyle ou alkoxy,

$R^3$ et $R^4$ représentent, indépendamment l'un de l'autre, l'hydrogène, un halogène ou un groupe alkyle,

$R^5$ et $R^6$ représentent, indépendamment l'un de l'autre, un halogène, un groupe nitro, amino, alkyle, perfluoralkyle, alkoxy, carboxyl, carboxylate alcalin, perfluoralkoxy ou perfluoralkylthio, et

$R^7$ est l'hydrogène, un halogène, un groupe X-A, phényle-X-A, alkyl-phényle-X-A ou $SO_2$-phényle-X-A, où

A est un équivalent d'une cation de métal alcalin ou représente

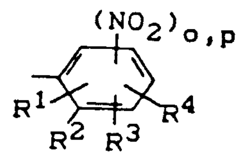

où o, p et $R^1$ à $R^4$ ont la définition indiquée et

$R^8$ et $R^9$ représentent, indépendamment l'un de l'autre, l'hydrogène, un halogène ou un groupe alkyle, et en outre, l'un des restes $R^3$ ou $R^4$ peut aussi représenter

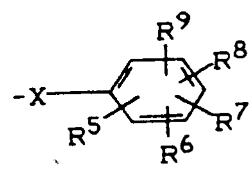

où X et $R^5$ à $A^9$ ont la définition indiquée, par réaction d'halogéno-nitrobenzènes de formule

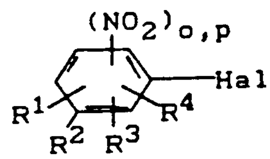 ,

dans laquelle

o, p et $R^1$ à $R^4$ ont la définition indiquée, avec des (thio)phénolates de formule

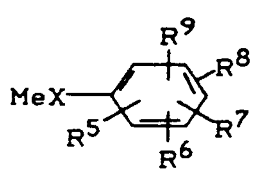 ,

dans laquelle

Me désigne un équivalent d'un métal alcalin et

X et $R^5$ à $R^9$ ont la définition indiquée, le groupe carboxyle pouvant toutefois se présenter sous la forme COOMe,

dans l'ammoniac liquide, caractérisé en ce qu'on conduit la réaction sous pression et à une température de 0 °C à +120 °C, de préférence de +10 °C à +110 °C et on chasse l'ammoniac par distillation lorsque la réaction est terminée.

**2.** Procédé suivant la revendication 1, caractérisé en ce qu'on fait réagir des halogéno-nitrobenzènes de formule

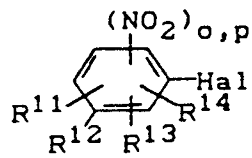

dans laquelle

o, p désignent la position ortho ou la position para du groupe nitro par rapport à l'halogène,

$R^{11}$ est l'hydrogène, le chlore, un groupe nitro, cyano, méthyle, éthyle ou trifluorométhyle,

$R^{12}$ est l'hydrogène, le chlore, le brome, un groupe méthyle, éthyle, trifluorométhyle, méthoxy ou éthoxy et

$R^{13}$ et $R^{14}$ représentent, indépendamment l'un de l'autre, l'hydrogène ou le chlore.

**3.** Procédé suivant la revendication 1, caractérisé en ce qu'on fait réagir des chloro-nitrobenzènes, de formule

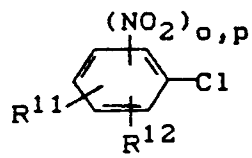

dans laquelle

o, p représentent la position ortho ou la position para du groupe nitro par rapport au chlore,

$R^{11}$ est l'hydrogène, le chlore, un groupe nitro, cyano, méthyle, éthyle ou trifluorométhyle et

$R^{12}$ est l'hydrogène, le chlore, le brome, un groupe méthyle, éthyle, trifluorométhyle, méthoxy ou éthoxy.

**4.** Procédé suivant la revendication 1, caractérisé en ce qu'on fait réagir des (thio)phénolates de formule

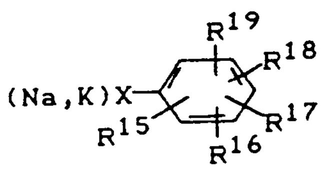

dans laquelle

X représente l'oxygène ou le soufre,

$R^{15}$ et $R^{16}$ représentent, indépendamment l'un de l'autre, l'hydrogène, le chlore, le brome, un groupe nitro, amino, méthyle, éthyle, trifluorométhyle, méthoxy ou éthoxy, un groupe carboxyle qui se présente sous la forme COONa ou COOK, un groupe trifluorométhoxy ou trifluorométhylthio,

$R^{17}$ est l'hydrogène, le chlore, un groupe X-$A^1$, phényle-X-$A^1$ ou alkyl-phényle-X-$A^1$ et

$R^{18}$ et $R^{19}$ représentent, indépendamment l'un de l'autre, l'hydrogène, le chlore, un groupe méthyle ou éthyle,

$A^1$ représentant Na, K ou le reste

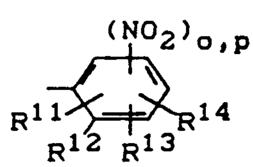

dans lequel

$R^{11}$ est l'hydrogène, le chlore, un groupe nitro, cyano, méthyle, éthyle ou trifluorométhyle,

$R^{12}$ est l'hydrogène, le chlore, le brome, un groupe méthyle, éthyle, trifluorométhyle, méthoxy ou éthoxy et

$R^{13}$ et $R^{14}$ représentent, indépendamment l'un de l'autre, l'hydrogène ou le chlore.

**5.** Procédé suivant la revendication 1, caractérisé en ce qu'on fait réagir des (thio)phénolates de formule

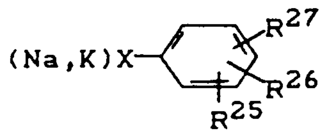

dans laquelle

X représente l'oxygène ou le soufre,

$R^{25}$ est l'hydrogène, le chlore, un groupe nitro, amino, méthyle, éthyle, trifluorométhyle, méthoxy, éthoxy, carboxyle, qui se présente sous la forme COONa ou COOK, trifluorométhoxy ou trifluorométhylthio,

$R^{26}$ est l'hydrogène, le chlore, le brome, un groupe méthyle, éthyle, carboxyle, qui se présente sous la forme COONa ou COOK, ou un groupe trifluorométhyle et

$R^{27}$ est l'hydrogène, le chlore ou un groupe -X-$A^2$,

$A^2$ représentant Na, K ou le reste

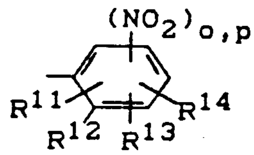

dans lequel

$R^{11}$ est l'hydrogène, le chlore, un groupe nitro, cyano, méthyle, éthyle ou trifluorométhyle et

$R^{12}$ est l'hydrogène, le chlore, le brome, un groupe méthyle, éthyle, trifluorométhyle, méthoxy ou éthoxy.

**6.** Procédé suivant la revendication 1, caractérisé en ce qu'on ajuste, pour chaque liaison (thio)éther diphénylique à former, un rapport molaire de l'halogéno-nitrobenzène au groupe (thio)phénolate de 0,7-1,2:1, de préférence de 0,9-1,05:1, notamment de 0,95-1:1.

**7.** Procédé suivant la revendication 1, caractérisé en ce qu'on utilise, par mole d'halogéno-nitrobenzène, 4 à 12 moles d'ammoniac liquide, de préférence 5 à 10 moles d'ammoniac liquide.

**8.** Procédé suivant la revendication 1, caractérisé en ce qu'on ajoute au mélange réactionnel, avant la séparation (totale) par distillation de l'ammoniac, un solvant inerte, de préférence le benzène, le toluène, le xylène, le chlorobenzène, le dichlorobenzène ou un hydrocarbure aliphatique tel que le cyclohexane ou l'isododécane, notamment le benzène, le toluène, le xylène ou un hydrocarbure aliphatique ou cycloaliphatique, on sépare l'halogénure alcalin après avoir chassé l'ammoniac et on recueille le produit réactionnel en le séparant du solvant inerte d'une manière connue.

**9.** Procédé suivant la revendication 1, caractérisé en ce qu'on ajoute de l'eau au mélange réactionnel après séparation par distillation d'une partie de l'ammoniac, on sépare la phase organique de la phase aqueuse après élimination de l'ammoniac restant et on traite finalement d'une manière connue la phase organique pour obtenir le produit réactionnel.

**10.** Procédé suivant la revendication 1, caractérisé en ce que le (thio)phénolate est formé dans le mélange réactionnel à partir de (thio)phénol et d'un hydroxyde de métal alcalin, le groupe carboxyle présent étant converti en même temps en carboxylate alcalin.